# EUROPÄISCHE PATENTANMELDUNG

(11) **EP 0 761 661 A1**
(43) Veröffentlichungstag der Anmeldung: **12.03.1997**
(21) Anmeldenummer: 96113616.5
(22) Anmeldetag: 26.08.1996
(51) Int. Cl.: C07D 307/20, C07D 309/10, A61K 31/34, A61K 31/35

(54) **Neuartige Glycomimetika als Selektin-Antagonisten und daraus hergestellte entzündungshemmend wirkende Arzneimittel**

(30) Priorität: 06.09.1995 DE 19532902
(71) Anmelder: HOECHST AKTIENGESELLSCHAFT, 65926 Frankfurt am Main (DE)
(72) Erfinder: Schmidt, Wolfgang, Dr., 65929 Frankfurt (DE); Sprengard, Ulrich, 65462 Gustavsburg (DE); Kretzschmar, Gerhard, Dr., 65760 Eschborn (DE); Kunz, Horst, Prof. Dr., 55127 Mainz (DE)

(57) **Zusammenfassung**

Die Erfindung betrifft neue Mimetika der Tetrasaccharide Sialyl Lewis-X (SleX) und Sialyl Lewis-A (SleA) mit verbesserter Wirkung als Inhibitoren der Zelladhäsion, nämlich eine Verbindung der Formel I 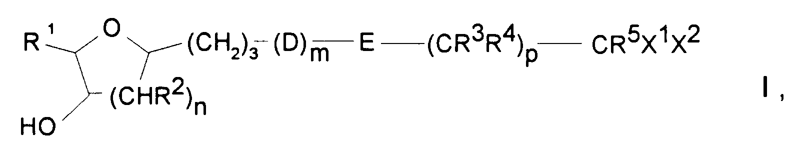 worin bedeuten
- R¹: -H, -CH₃, oder -CH₂OH,
- R²: -H oder -OH,
- R^{3,} R⁴ und R⁵: unabhängig voneinander -H, C₁-C₄-Alkyl oder -OH,
- R⁶, R⁷, R⁸, R⁹ und R¹⁰: unabhängig voneinander -H oder C₁-C₄-Alkyl,
- D: -O-C(O)-, -C(O)- oder -NR⁶-C(O)-,
- E: -CR⁷R⁸-, -NR⁷-, einen Stickstoffheterozykus der Formel 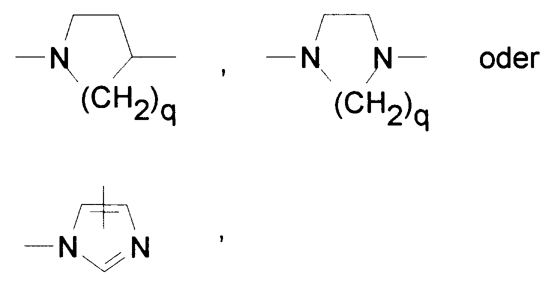
- n: 1 oder 2,
- m: 0 oder 1
- p: eine ganze Zahl von 0 bis 10,
- q: 1 oder 2 und
- X¹ und X²: unabhängig voneinander -H, -COOR⁹, -NR⁹R¹⁰, -OH, -OSO₃H, -CH₂COOR⁹, -CH₂OSO₃H oder gemeinsam = O,
ein Verfahren zur Herstellung dieser Verbindungen sowie deren Verwendung als pharmakologische Wirkstoffe und Diagnostika.

## Beschreibung

Die Erfindung betrifft neue Mimetika der Tetrasaccharide Sialyl Lewis-X (SLeX) und Sialyl Lewis-A (SLeA) mit verbesserter Wirkung als Inhibitoren der Zelladhäsion, ein Verfahren zur Herstellung dieser Verbindungen sowie deren Verwendung als pharmakologische Wirkstoffe und Diagnostika.

Die Zirkulation von Blutzellen wie z.B Leukozyten, Neutrophilen, Granulozyten und Monozyten ist auf molekularer Ebene ein vielstufiger, sehr komplexer Prozess, der nur in Teilschritten bekannt ist (Review: T.A.Springer, Cell 76, 301-314, 1994).

Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.

An diesem Vorgang sind zahlreiche spezifische Signalmoleküle wie z.B. Interleukine, Leukotriene und Tumornekrosefaktor (TNF), deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die in der Anfangsphase entzündlicher Prozesse durch Selektin-Rezeptoren vermittelte Adhäsion von Leukozyten an Endothelzellen ist eine natürliche und notwendige Immunantwort auf diverse Entzündungsreize und Verletzungen des vasculären Gewebes.

Der Verlauf einer Reihe von akuten und chronischen Erkrankungen wie beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie und By-Pass Operationen) auftretende Restenose wird jedoch durch die übermäßige Adhäsion von Leukozyten und deren Infiltration in das betroffene Gerwebe ungünstig beeinflußt. Die Beeinflussung dieser Adhäsionsprozesses in einem sehr frühen Stadium der Entzündung ist deshalb ein sehr attraktives und generell anwendbares Konzept zur pharmakologischen Kontrolle entzündlicher Erkrankungen.

Es wird heute generell anerkannt, daß die Tetrasaccharide Sialyl Lewis-X (SLeX) und Sialyl Lewis-A (SLeA), die als Teilstrukturen von Glycosphingolipiden und Glycoproteinen auf Zellmembranen vorkommen, als Liganden für alle drei Selektinrezeptoren fungieren können.
In der Literatur werden eine Reihe von Glycoproteinen, Mucinen und Glycolipide beschrieben, die als endogene Liganden der Selektine in Frage kommen. Dazu gehören das Mucosal Vascular Addressin MadCAM-1 (Berg et.al., Nature 1993, 366, 695) und das Sialomucin CD34 (Baumhuter et al., Science 1993, 262, 436) für L-Selektin, ein O-linked Polylactosamin-Sialomucin PSGL-1 auf humanen Neutrophilen für P-Selektin (Moore et.al., J.Biol.Chem. 1994, 269, 23318) und N-linked Sialoglycoproteine vom Typ ESL-1 für E-Selektin (Vestweber et.al., Cell Biol. 1993, 121, 449).

Die Spezifität dieser und anderer potentieller Liganden für Selektine in vivo ist noch nicht geklärt. Die auf Selektin-Liganden vorkommenden Tetrasaccharide SLeX und SLeA repräsentieren lediglich eine Teilstruktur der wesentlich komplexeren endogenen Ligandenstrukturen und können aufgrund ihrer sehr ähnlichen Affinität zu Selektinen nicht allein zur Erklärung einer spezifischen Rezeptorbindung herangezogen werden.

Aufgrund der Komplexität dieser Strukturen ist der Versuch, die an Selektine bindende Tetrasaccharide SLeX/A als Teilstrukturen in verschiedenen Darreichungsformen oder einfache Mimetika derselben mit modifizierter Struktur als Antagonisten zur Modulierung oder Unterbindung einer übermäßigen Leukozytenadhäsion einzusetzen und so zur Linderung bzw. Heilung genannter Erkrankungen beizutragen, ein vielversprechender therapeutischer Ansatz.

Der natürliche Ligand mit der Struktur von SLeX wurde schon erfolgreich in Tierversuchen bei P-Selektin abhängigen Lungenverletzungen (M.S.Mulligan et.al., Nature 1993, 364, 149) und bei myokardialen Reperfusionsverletzungen (M.Buerke et.al., J.Clin.Invest. 1994, 93, 1140) verwendet. In ersten klinischen Prüfungen bei akuter Lungenentzündung soll die Verbindung in einer Dosis von 1-2 Gramm pro Tag und Patient eingesetzt werden (Mitteilung der Firma Cytel Corp,/ La Jolla (CA.) beim 2. Glycotechnology Meeting/CHI in La Jolla/USA am 16.-18. Mai 1994).

In einigen Publikationen und Patentanmeldungen wurde inzwischen über Bemühungen berichtet, durch strukturelle Variation des Liganden zu fester bindenden Antagonisten zu gelangen. Ziel dieser Arbeiten ist die Bereitstellung wirksamerer Antagonisten, die auch potentiell in vivo bei geringerer Dosis einsetzbar wären.

Die Variation der für die Struktur-Wirkungs-Beziehung bislang als entscheidend angesehenen Fucose- und Neuraminsäurebausteine (B.K.Brandley et.al., Glycobiology 1993, 3, 633 und M.Yoshida et.al., Glycoconjugate J. 1993, 10, 3) erbrachte jedoch keine signifikant verbesserten Inhibitionswerte. Allein bei Variation des Glucosaminbausteins (Ersatz von GlcNAc durch Glucose und Azido- sowie Aminogruppen in der 2-Position von GlcNAc) ließ sich eine signifikant erhöhte Affinität an den E-Selektin-Rezeptor erreichen. Beim P-Selektin-Rezeptor wurde hingegen keine verbesserte Bindung erreicht.

Generell blieben bisher alle Erfolge zur Verbesserung der Bindungsaffinität von SLeX-und SLeA-Derivaten auf den E-Selektinrezeptor beschränkt, denn mit dem P-Selektinrezeptor wurden nur schwache Inhibitionseffekte bei Inhibitorkonzentrationen von ca. 1 mM gefunden (R.M.Nelson et.al., J.Clin.Invest. 1993, 91, 1157).

Der Stand der Technik zur Bindungsaffinität modifizierter SLeX/A-Strukturen an Selektine wird in Pharmacochem. Libr. 1993, 20 (Trends in Drug Research), Seiten 33-40 referiert.

Neben der geringen Bindungsaffinität an die Selektine, die diese Verbindungen aufweisen, enthalten sie jedoch alle mindestens eine labile glycosidische Bindung, was die orale Verfügbarkeit dieser Wirkstoffe extrem einschränkt. Diese Labilität schränkt auch den Aufbau von verschiedenen Derivaten stark ein, da die empfindliche, zur Spaltung neigende glycosidische Bindung die Reaktionsbedingungen stark einschränkt. Verschiedenste Ansätze der Synthese von Mimetika wurden bearbeitet, um zu einer Erhöhung der Stabilität zu gelangen.

Eine Erhöhung der Stabilität erfolgte durch die Anknüpfung der Seitenkette über eine C-C Verknüpfung an dem Kohlenstoff C-4 der Fucose (Floyd et al, Tetrahedron Asymmetry 1994, 5, 2061).

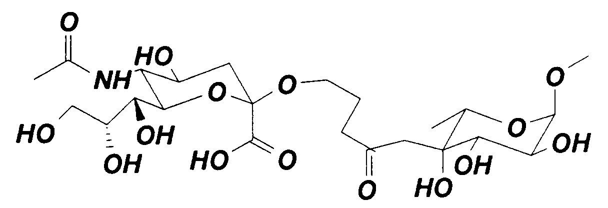

Hierbei weicht jedoch durch die Anknüpfung an C-4 der Fucose die Orientierung der Seitenkette von der des natürlichen Liganden ab. Es zeigte sich nur eine sehr geringe Affinität zur Bindung an die Selektine.

Der Einsatz carbacyclischer Kohlenhydratanaloga, bei der die Anknüpfung der Seitenkette durch eine C-C Bindung an C-1 erfolgt, würde eine dem natürlichen Liganden ähnliche Konformation aufweisen, aber trotzdem gegen Abbau stabil sein. Mit großer Intensität wird an der Darstellung carbacyclischer Kohlernhydratanaloga von Monosaccharidbausteinen gearbeitet.

Von den verschiedenen Methoden sind die Umsetzungen von aktivierten Monosacchariden mit Nitromethan (Gross P.H., Tetrahedron 1991, 47,6113), Allylsilan (Y. Kishi et. al., J. Am. Chem. Soc. 104, 4976-4978, 1982) und Olefinen (D.A. Levy et. al., Tetrahedron Asymmetry 5, 2265-2268, 1994) hervorzuheben, die sich aufgrund der eingeführten Funktionalität als Baustein für weitere Kupplungen eignen.

Die Nutzung eines carbacyclischen Analogons als Baustein für Selektinantagonisten führte zu einem Mimetikum mit Affinität an Selektine. Hierbei konnte durch Umsetzung eines Fucosebausteines mit Allylsilan ein spezieller C-glycosidischer Baustein **1** mit α-Orientierung der Seitenkette aufgebaut werden (WO 95/04751). Die Selektivitäten in der Allylierung sind mit α/β = 14/1 sehr gut, jedoch ist ein up-scaling der Reaktion aufgrund der vorgeschlagenen Reaktionsbedingungen nicht einfach möglich. Ebenfalls ist eine chromatographische Reinigung des Bausteines erforderlich, bei der sich das α/β Gemisch jedoch nicht trennen läßt.

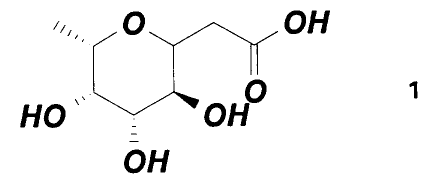

Die endständige Säurefunktion der Seitenkette wird zum Aufbau von Glycopeptidanaloga z.B. **2** eingesetzt. Die Derivate weisen IC₅₀-Werte im Bereich von bis zu 1 mM auf. Die Glycopeptidanaloga sind jedoch gegen den Abbau der Peptidkette durch Proteasen nicht stabil, so daß die orale Verfügbarkeit dieser Verbindungen, obwohl der Zuckerbaustein durch das C-Glycosid stabil geworden ist, weiterhin stark eingeschränkt ist. Ein weiterer Nachteil ist die β-Verbindung, die bei dieser Synthese nicht abgetrennt werden kann. Die entsprechenden β-Derivate weisen keine Wirksamkeit auf, da die Seitenkette falsch orientiert wird, wie entsprechende Modellrechnungen belegen.

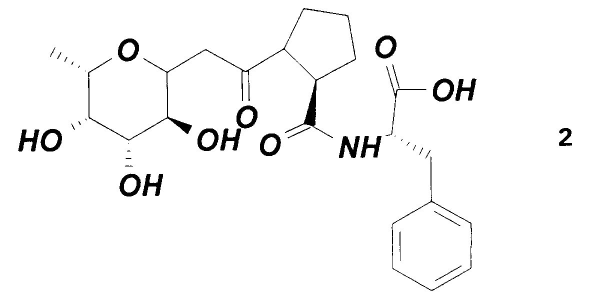

In analoger Weise wurde der C-glycosidische Baustein 1 ebenfalls zum Aufbau verschiedener weiterer Mimetika eingesetzt, die die aktive Konformation des SLe^{x} imitieren sollten. Die getesteten Verbindungen z.B. **3** zeigten jedoch mit 10-20 mM um den Faktor 10-20 höhere IC₅₀-Werte als der natürliche Ligand SLe^{x} (Wong, et. al., J. Am. Chem. Soc. 1995, 117, 5395).

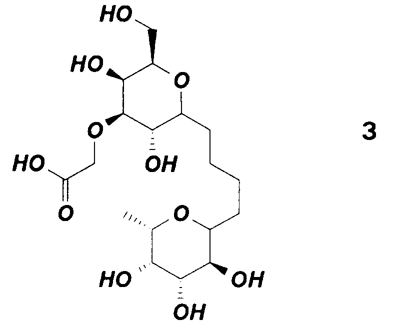

Durch den Einsatz eines substituierten Allylsilans konnte das C-Glycosid **4** dargestellt werden.

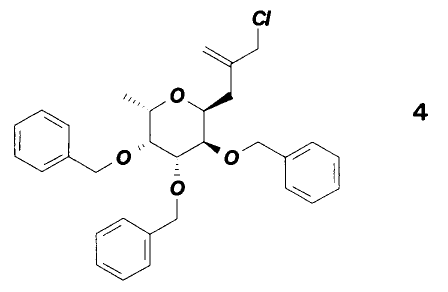

Durch Anknüpfung an Triterpenoid Säurederivate (Betulinic Acid: WO 95/04526; Glycyrrhetinic Acid: WO 94/24145) 4a wurden Mimetika dargestellt, die eine "multi-medicament capacity" aufweisen sollen und in verschiedensten Testsystemen getestet wurden (Inhibierung von 5-Lipoxygenase, Antimetastatische Wirkung, P-Selektin Hemmung). Hierbei ergab sich für die Bindung an P-Selektin ein IC₅₀-Wert von 0.75 mM. Zu beachten ist hierbei jedoch, daß allein die Triterpenoid Säure in diesem Test schon Werte von 0.125 mM aufweist.

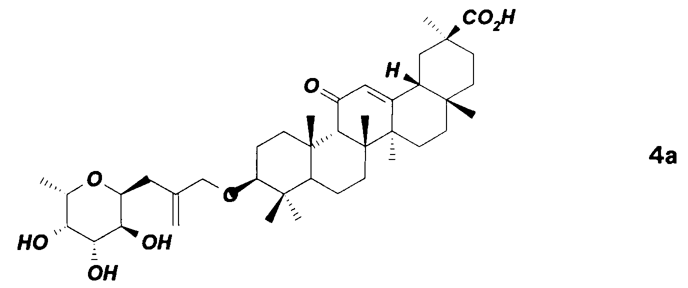

Die Darstellung des C-Glycosidbausteines **4** erfordert ebenfalls eine aufwendige chromatographische Reinigung, bei der das β-Derivat nur schwer abgetrennt werden kann. Auch die Stabilität des C-Glycosids ist durch die reaktive Allylchlorid-Gruppierung eingeschränkt.

Die genannten C-Glycosid Mimetika weisen neben den bereits beschriebenen präparativen Problemen (aufwendige chromatographische Reinigung, hohe Stufenzahl durch Schutzgruppenstrategie, α/β-Gemisch) eine zu geringe Bindungsaffinität an die Selektine auf, um in die Adhäsionsprozesse wirksam eingreifen zu können. Denn neben dem Aspekt der reinen Stabilität eines Fucose-Mimetikums ist für die Bindung an die Selektine vor allem auch die Ausrichtung und konformative Fixierung der Seitenkette von besonderer Bedeutung, die die genannten Derivate nicht erfüllen. Dieser Aspekt wird besonders bei den Derivaten von Wong et. al. deutlich, wo geringfügige Änderungen in der Struktur der Seitenkette deutliche Auswirkungen auf die Bindungsstärke aufweisen (Austausch O - C).
Nach L.A. Lasky ist die negativ geladene Sialinsäure (oder eine negativ geladenen Sulfonsäuregruppe) absolut notwendig für die Bindung an Selektine. Da die Kristallstruktur von E-Selektin aufgeklärt werden konnte, wurden bereits Untersuchungen über mögliche Bindungsstellen durchgeführt. Als mögliche Bindungstellen für die Sialinsäurefunktion wurden hierbei zum einen die beiden Lysine K111 bzw. K113 (J.Bajorath et.al., Biochemistry 1994, 33, 1332) und zum anderen Arg 97, Lys 111 und Lys 113 (Structural Biology 1, 140 (1994)) vorgeschlagen.

Demgegenüber ist es Aufgabe der vorliegenden Erfindung, stabile, niedermolekulare Mimetika der Sialyl-Lewis-X bzw. Sialyl-Lewis-A-Strukturen bereitzustellen, deren Konstitution und Konfiguration eine signifikant gesteigerte Affinität an Selektine besitzen, synthetisch einfacher als Oligosaccharide zugänglich sind und strukturbedingt als potentiell oral verfügbare Arzneinmittel geeignet sind.

Die gestellte Aufgabe wird gelöst durch eine Verbindung der Formel I 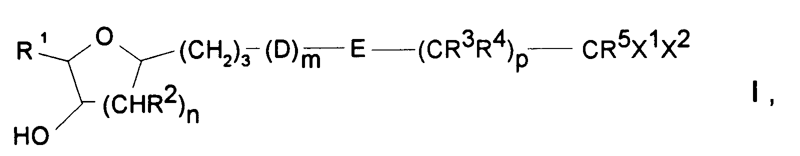 worin bedeuten
- R¹: -H, -CH₃, oder -CH₂OH,
- R²: -H oder -OH,
- R^{3,} R⁴ und R⁵: unabhängig voneinander -H, C₁-C₄-Alkyl oder -OH,
- R⁶, R⁷, R⁸, R⁹ und R¹⁰: unabhängig voneinander -H oder C₁-C₄-Alkyl,
- D: -O-C(O)-, -C(O)- oder -NR⁶-C(O)-,
- E: -CR⁷R⁸-, -NR⁷-, einen Stickstoffheterozykus der Formel 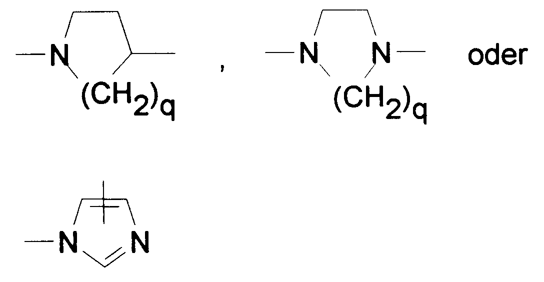
- n: 1 oder 2,
- m: 0 oder 1,
- p: eine ganze Zahl von 0 bis 10,
- q: 1 oder 2 und
- X¹ und X²: unabhängig voneinander -H, -COOR⁹, -NR⁹R¹⁰, -OH, -OSO₃H, -CH₂COOR⁹, -CH₂OSO₃H oder gemeinsam =O.

Bevorzugt ist die Verbindung der Formel I dadurch gekennzeichntet, daß m 1 ist und D -O-C(O)- bedeutet, oder dadurch gekennzeichnet, daß m 0 ist.

Wahlweise bedeuten in Formel I die Variablen n 2 und R² -OH, wobei R¹ vorzugsweise für -CH₃ oder -CH₂OH steht, besonders bevorzugt weist der Oxazyklus in Formel I 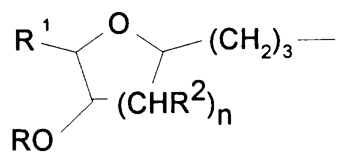 die Konfiguration eines L-Fucosylrestes oder eines D-Mannosylrestes auf, oder es bedeuten n 1, R¹ -CH₂OH und R² -OH, wobei der Oxazyklus in Formel I besonders bevorzugt die Konfiguration eines Ribosylrestes aufweist. Die Verbindung gemäß der vorliegenden Erfindung kann ferner wahlweise dadurch gekennzeichnet sein, daß
1.
   - E: -NR⁷-,
   - R⁷: -H,
   - p: 0,
   - R⁵: -H
   - X¹: -COOR⁹,
   - X²: -CH₂COOR⁹ und
   - R⁹: -H bedeuten,
   - beispielsweise: 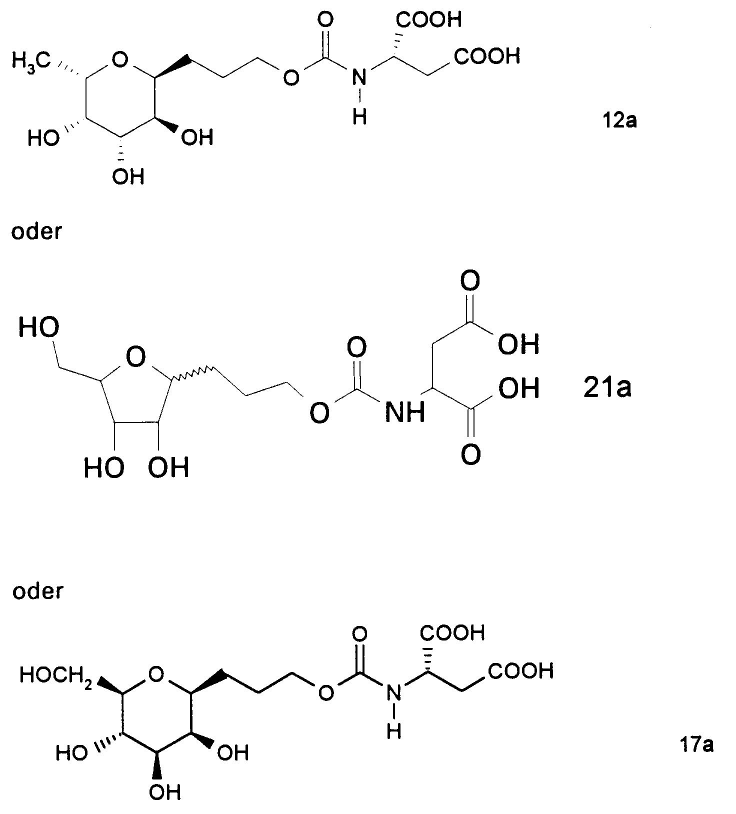
2. oder dadurch gekennzeichntet, daß
   - E: -NR⁷-,
   - R⁷: -H,
   - p: 4,
   - R³ und R⁴: -H
   - R⁵: -H
   - X¹: -COOR⁹,
   - X²: -NR⁹R¹⁰ und
   - R⁹ und R¹⁰: -H bedeuten,
   - beispielsweise: 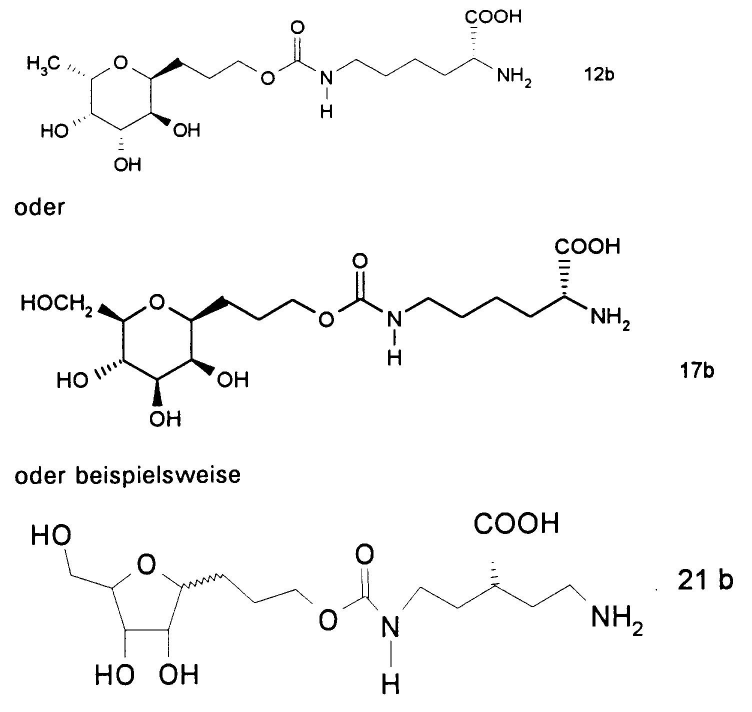
3. oder dadurch gekennzeichntet, daß
   - E: einen Heterozyklus der Formel 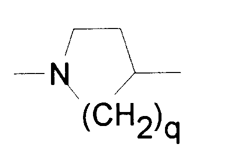 darstellt,
   - q: 2,
   - p: 0,
   - R⁵: -OH und
   - X¹ und X²: gemeinsam = O bedeuten, beispielsweise 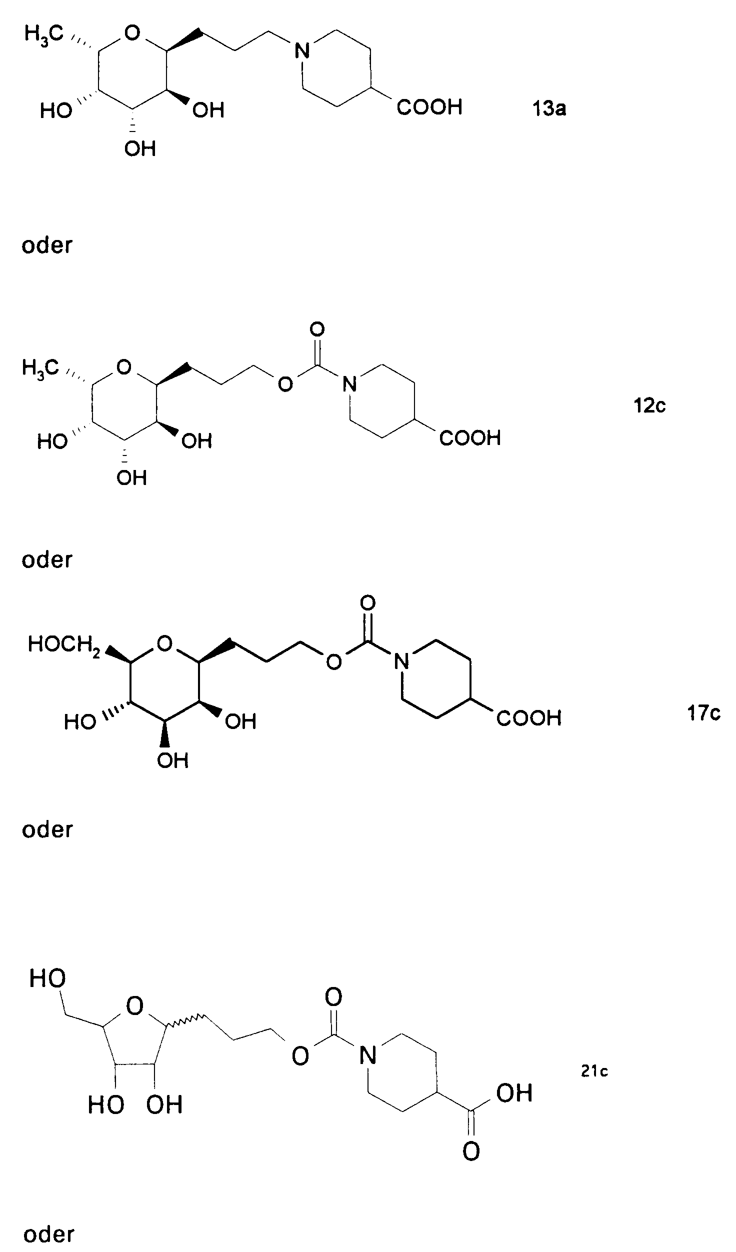 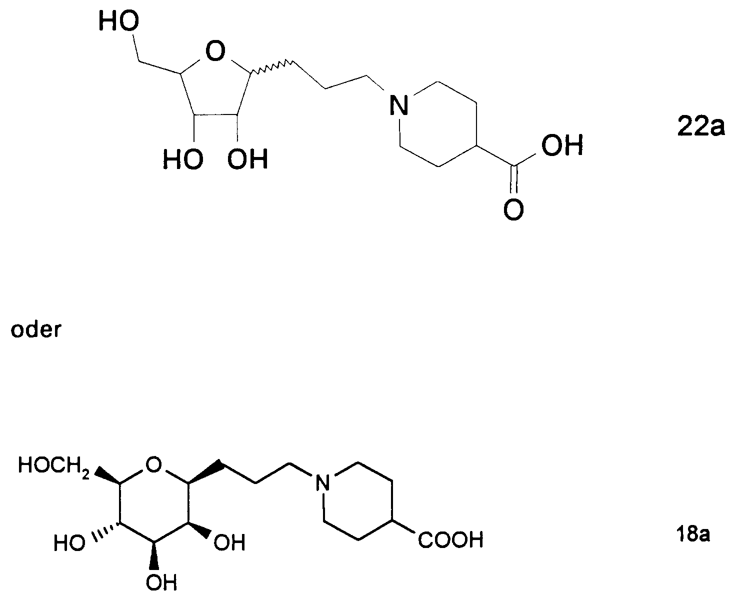
4. oder dadurch gekennzeichntet, daß
   - E: einen Hetreozyklus der Formel 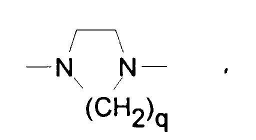 darstellt,
   - q: 2,
   - p: 0,
   - R⁵ und R⁹: -H und
   - X¹ und X²: -COOR⁹ bedeuten, beispielsweise

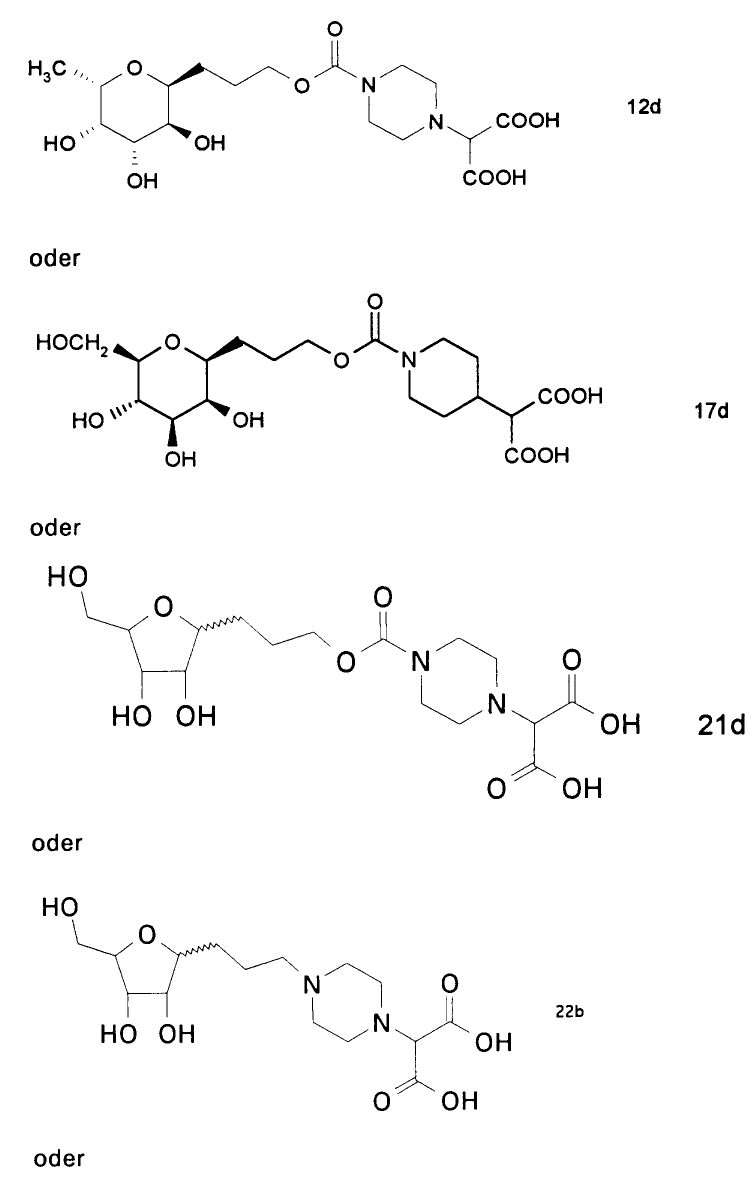

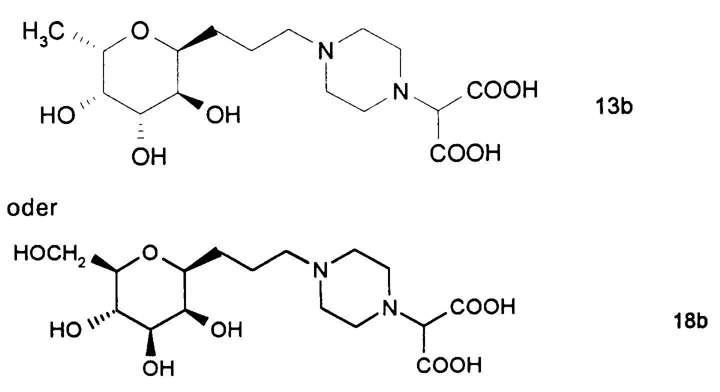

Die Aufgabe wird ferner gelöst durch ein Verfahren zur Herstellung einer Verbindung der Formel I, welches sich dadurch auszeichnet, daß man eine Verbindung der Formel II 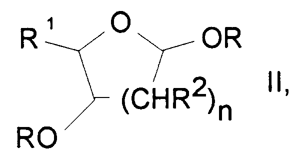 worin R¹, R² und n die genannten Bedeutungen haben, wobei R¹ und R² notwendigenfalls mit Schutzgruppen versehen sind, und R eine Acetylgruppe bedeutet, unter der Wirkung eines Lewissäurekatalysators in einem geeigneten Lösungsmittel, beispielsweise Bortrifluoridetherat in Acetonitril oder Trimethylsilyltrifuormethansulfonat (Trimethylsilyltriflat) in Nitromethan, mit einem Allylsilan zu einer Verbindung der Formel III 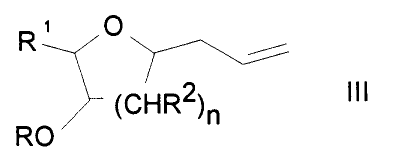 umsetzt, in welcher sämtliche Variablen die genannten Bedeutungen haben und welche sich in der Regel durch einfache Umkristallisation rein darstellen läßt (d.h. ohne Beimengung des entsprechenden Epimers), worauf nach Umwandlung sämtlicher Schutzgruppen in Benzylschutzgruppen und unter Funktionalisierung der allylischen Doppelbindung eine Verbindung der Formel IV 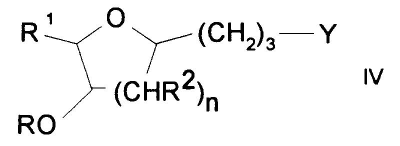 hergestellt wird, in welcher die Variblen R¹ und R² die genannten Bedeutungen haben und notwendigenfalls mit Schutzgruppen versehen sind, R eine Benzylgruppe bedeutet und Y Halogen oder eine beispielsweise als Aktivester aktivierte OH-Gruppe darstellt, wonach die Verbindung der Formel IV mit einer Verbindung der Formel V

X―E―(CR³R⁴)ₚ―CR⁵X¹X² V

umgesetzt wird, inwelcher X -H oder -COOH bedeutet und sämtliche übrigen Variablen die genannten Bedeutungen haben, worauf man nach Abspaltung sämtlicher Schutzgruppen eine Verbindung der Formel I erhält.

Die aktivierte OH-Gruppe Y läßt sich vorteilhafterweise durch Umsetzung der freien OH-Gruppe mit Chlorameisensäure(p)-nitrophenylester erzeugen (Polymer 34, 26-33 (1993)).

Die Verbindungen gemäß der vorliegenden Erfindung sowie deren physiologisch vertraglichen Salze eignen sich aufgrund ihrer wertvollen pharmakologischen Eigenschaften sehr gut zur Anwendung als Heilmittel bei Säugern, insbesondere dem Menschen.

Die vorliegende Erfindung betrifft daher ferner ein Arzneimittel enthaltend die Verbindung der Formel I sowie deren Verwendung für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen, beispielsweise Rheuma, Reperfusionsverletzungen, Ischämie oder Herzinfarkt. Die Arzneimittel eignen sich im besonderen zur Behandlung von akuten und chronischen Entzündungen, die sich pathophysiologisch durch eine Störung der Zellzirkulation, beispielsweise von Lymphozyten, Monozyten und neutrophilen Granulozyten, kennzeichnen lassen. Hierzu zählen Autoimmunerkrankungen wie akute Polyarthritis, rheumatoide Arthritis und Insulin-abhängige Diabetes (Diabetes mellitus IDDM) akute und chronische Transplantatabstoßungen, Schocklunge (ARDS, adult respiratory distress syndrome), entzündliche und allergische Hauterkrankungen wie beispielsweise Psoriasis und Kontakt-Ekzeme, Herz-Kreislauf-Erkrankungen wie Myokardinfarkt, Reperfusionsverletzungen nach Thrombolyse , Angioplastie oder By-Pass-Operationen, septischer Schock und systemischer Schock. Eine weitere potentielle Indikation ist die Behandlung metastasierender Tumoren, denn Tumorzellen tragen Oberflächenantigene, die sowohl Sialyl-Lewis-X als auch Sialyl-Lewis-A-Strukturen als Erkennungsepitope besitzen. Darüberhinaus können diese Arzneimittel, die stabil im sauren Milieu des Magens sind, zur antiadhäsiven Therapie von Helicobacter Pylori und verwandten Mikroorganismen ggf. auch in Kombination mit Antibiotika eingesetzt werden. Ferner ist mit Hilfe dieser Arzneimittel eine Therapie der cerebralen Form der Malaria denkbar.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche exprimiert werden und demzufolge die zu verabreichende Tagesdosis geringer ist als bei stark erkrankten Patienten.

Die erfindungsgemäßen Arzneimittel werden dadurch hergestellt, daß man eine Verbindunmg gemäß der vorliegenden Erfindung mit üblichen Träger- sowie gegebenenfalls Zusatz- und/oder Hilfsstoffen in die bzw. eine geeignete Darreichungsform bringt.

Im folgenden wird die Erfindung detailliert beschrieben, insbesondere in ihren bevorzugten Ausführungsformen.

### Synthese von Verbindungen der Formel I

Die Synthese des reinen α-C-Glycosids **8** konnte in nur 3 Stufen mit einer Gesamtausbeute von 72 % durchgeführt werden (Schema).

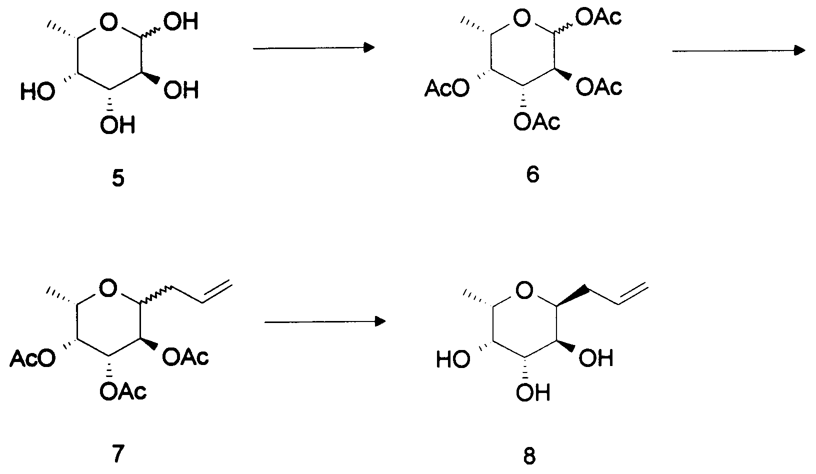

Nach Acetylierung von L-Fucose **5** mit Acetanhydrid/Pyridin (95-98 %) wurde unter BF₃-Katalyse mit einem Allylsilan umgesetzt (92 %, α/β Verhältnis 10/1). Das erhaltene C-Glycosid **7** wurde entacetyliert (quantitativ) und durch Umkristallisation gereinigt. Durch geeignete Wahl der Umkristallisationsbedingungen konnte das α-Derivat **8** im Gegensatz zu bekannten Verfahren rein erhalten werden (d.h. ohne Beimengungen des entsprechenden β-Derivate). Den einzigen Reinigungsschritt in dieser Sequenz stellt die Umkristallisation dar.

Das C-Glycosid **8** kann zur Funktionalisierung und Fixierung der Seitenkette eingesetzt werden.
Schutz der Hydroxylgruppen in **8** durch Benzylierung und anschließende Hydroborierung mit Boran liefert den Alkohol **10** in 63 % Ausbeute. Der Alkohol wird mit Triphenylphosphin/Dibromtetrachlorethan mit 92 % Ausbeute in das Bromid **11** überführt.

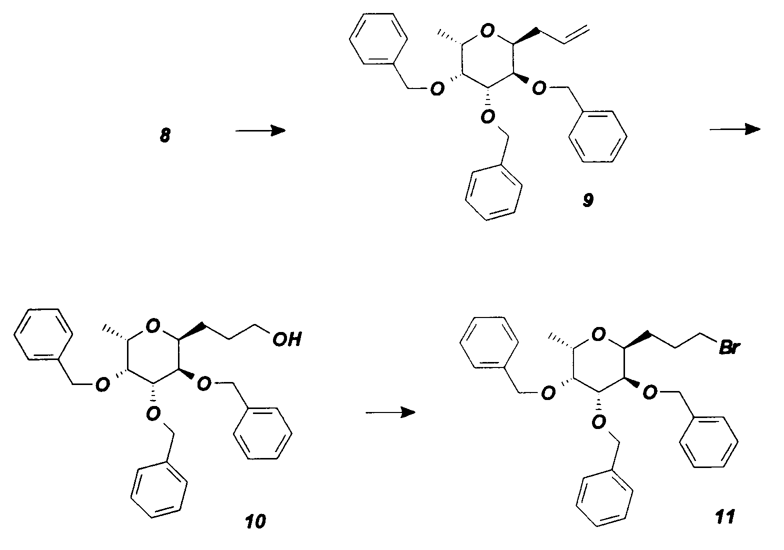

Der Alkohol **10** und das Bromid **11** sind geeignete Bausteine, um die für die Bindung erforderlichen Seitenkette aufzubauen ( s. Beispiele 12 a-d und 13a,b).

In analoger Weise lassen sich die Reaktionen auf andere C-Glycosidbausteine wie beispielsweise Verbindung 14, welche aus D-Mannose hergestellt werden kann, oder Verbindung 20, welche ausgehend von Ribose über Verbindung 19 herstellbar ist, übertragen.

Ferner lassen sich nach dem Verfahren gemäß der vorliegenden Erfindung entsprechend Derivate der L-Galactose, L-Rhamnose und der Glucose herstellen.

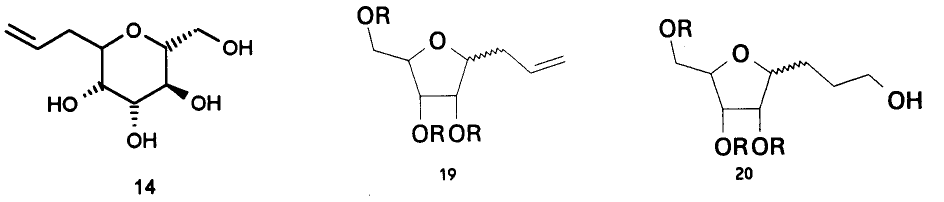

Primärassays zur Untersuchung der Wirkung der Verbindungen gemäß der vorliegenden Erfindung auf die Zellanheftung an rekombinante, lösliche Selektin-Fusionsproteine.

Um die Wirksamkeit der erfindungsgemäßen Verbindungen auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### Herstellung von L-Selektin-IgG1

Zur Herstellung von löslichem L-Selektion-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F. M., Brent, R., Kingston, R. E., Moore, D. D., Seidman, J. G., Struhl, K. und Smith, J. A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben. (Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.)

### Herstellung von P-Selektin-IgG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo, A., Kolanus, W., Walz, G., Fredman, P. und Seed, B. 1991. CD62/-P-Selectin recognition of myeloid and tumor cell sulfatides. Cell 67, 35-44.

### Herstellung von CD4-IgG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1. (Zettelmeissl, G., Gregersen, J.-P., Duport, J. M., Mehdi, S., Reiner, G. und Seed, B. 1990. Expression and characterization of human CD4: Immunoglobulin Fusion Proteins. DNA and Cell Biology 9, 347-353.)

### Durchführung des HL60 Zelladhäsionsassays auf rekombinanten, löslichen Adhäsionsmolekülen

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl2; 1 mM CaCl2; 3 mM MnCl2; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl2; 1 mM CaCl2; 3 mM MnCl2; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Leukozyten-Adhäsion - Prüfung der Wirksamkeit der erfindungsgemäßen Verbindungen in vivo

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune System. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301 - 314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphonuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tierversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspectives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation aus Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01. 1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (in Prozenten).

### Beispiele

### Beispiel 1

### Darstellung der C-Glycosidbausteine 10 und 11

### Acetylierung von Fucose

L-Fucose 5 (250 g, 1.52 Mol) wird mit Pyridin (616 ml, 7.62 Mol) und Acetanhydrid (633 ml, 6.7 Mol) versetzt und 24 h bei Raumtemperatur gerührt. Das Lösungsmittel wird am Rotationsverdampfer entfernt und der Rückstand am Hochvakuum getrocknet. Man erhält 6 (496 g, 98 %) als gelbes Öl.
DC [Hexan/Essigsäureethylester : 1/1]: R_{f} = 0.60. - ¹H-NMR (300 MHz, CDCl₃): d = 1.18 (d, 3 H, J_{6,5} = 6.9 Hz, 6-H^{Fuc}), 2.00-2.15 (4 s, 12 H, CH₃),

Das Fucosederivat **6** (50 g, 0.15 Mol) wird unter Argon in Acetonitril (300 ml) gelöst und auf -10 °C abgekühlt. Nach Zugabe von Allylsilan (47.9 ml, 0.30 Mol) und Bortrifluorid - Diethylether Komplex (20.4 ml, 0.166 Mol) wird das Gemisch 1 h bei -10 °C gerührt. Nach Erwärmen auf Raumtemperatur wird weitere 3 h gerührt. Die Lösung wird auf gesättigte Natriumhydrogencarbonatlösung gegeben und mit Essigester extrahiert. Das Lösungsmittel wird am Rotationsverdampfer entfernt. Der verbleibende Rückstand wird in Dichlormethan aufgenommen und über eine kurze Kieselgelsäule filtriert. Nach Entfernen des Lösungsmittels erhält man **7** (*a*/β 10/1) als gelbes Öl (43.5 g, 92 %).
DC [Hexan/Essigsäureethylester : 1/1]: R_{f} = 0.65 - ¹H-NMR (300 MHz, CDCl₃): d = 1.19 (d, 3 H, J_{6,5} = 6.9 Hz, 6-H^{Fuc}), 2.00-2.15 (3 s, 9 H, CH₃), 2.20-2.58 (m, 2H), 3.98 (m, 1H), 4.28 (m, 1H), 5.05-5.85 (m, 6H).

Das Allylderivat **7** (43.5 g, 0.139 Mol) wird in Methanol (200 ml) gelöst und mit Natriummethanolatlösung (3 ml, 30 %ig) versetzt. Die Lösung wird bei Raumtemperatur 2 h gerührt, mit saurem Ionenaustauscher (Dowex 50 W X 8) neutralisiert. Der Ionenaustauscher wird abfiltriert und das Lösungsmittel am Rotationsverdampfer abgezogen. Man erhält das entacetylierte Fucosederivat als leicht gelben Feststoff (26.0 g, quantitativ).
Der Rückstand wird in Essigester in der Wärme gelöst und heiß filtriert. Nach Entfernen des Lösungsmittels am Rotationsverdampfer wird der Rückstand in feuchtem Essigester in der Wärme erneut gelöst. Beim Abkühlen der Lösung kristallisiert **8** als weißer Feststoff aus (20.8 g, 80 %).
DC [Dichlormethan/Methanol: 10/1]: R_{f} = 0.25 - ¹H-NMR (300 MHz, D₂O): d = 1.05 (d, 3 H, J_{6,5} = 6.9 Hz, 6-H^{Fuc}), 2.19-2.43 (m, 2H), 3.60-4.00 (m, 5 H), 5.05 (m, 2 H), 5.62-5.80 (m, 1H).

Zu einer Suspension von Natriumhydrid (95 %ig, 2.27 g, 95 mmol ) in DMF (40 ml) unter Argon wird bei 0° C eine Lösung von **8** (5.0 g, 27,0 mmol) in DMF (5 ml) langsam zugetropft. Das Gemisch wird 30 Minuten bei dieser Temperatur gerührt. Tropfenweise wird Benzylbromid (10.3 ml, 86.5 mmol) zugegeben. Das Gemisch wird nach 1 h auf Raumtemperatur erwärmt und 12 h bei Raumtemperatur weiter gerührt. Nach vorsichtiger Zugabe von Methanol und Wasser wird mit Essigester extrahiert. Die organische Phase wird mehrfach mit Wasser gewaschen und über Magnesiumsulfat getrocknet. Entfernen des Lösungsmittels am Rotationsverdampfer ergibt einen öligen Rückstand der flashchromatographisch (Isohexan/Esssigester 4/1 - 1/1) gereinigt wird. Man erhält **9** (9.1 g, 19.9 mmol, 74 %) als farbloses Öl:
DC [Toluol/Essigester: 1/1]: R_{f} = 0.55 - ¹H-NMR (300 MHz, CDCl₃): d = 1.15 (d` 3 H, J_{6,5} = 6.9 Hz, 6-H^{Fuc}), 2.22-2.45 (m, 2H), 3.78 (m, 3 H), 3.90-4.10 (m, 2H), 4.40-4.80 (m, 6 H), 5.00-5.12 (m, 2H), 5.80 (m, 1H), 7.30-7.40 (m, 15 H).

Die Allylverbindung **9** (9.1g, 19.9 mmol) wird unter Argon in THF (40 ml) gelöst, auf 0°C abgekühlt und bei dieser Temperatur tropfenweise mit einer 1 M Boran -THF (25 ml) Lösung versetzt. Die Lösung wird nach beendeter Zugabe auf Raumtemperatur erwärmt und weitere 3 h gerührt. Das Gemisch wird erneut auf 0°C abgekühlt und vorsichtig mit 5 M NaOH - Lösung (25 ml) und 30 %iger Wasserstoffperoxidlösung (20 ml) versetzt. Nach Extraktion mit Essigsäureethylester wird das Lösungsmittel am Rotationsverdamper entfernt. Der Rückstand wird flashchromatograpisch (Isohexan/Esssigester 2/1 - 1/1) gereinigt. Man erhält **10** (8.05 g, 16.9 mmol, 85 %) als farbloses Öl.

Der Alkohol **10** (740 mg, 1.6 mmol) wird in Dichlormethan (10 ml) gelöst und die Lösung auf 0°C abgekühlt. Nacheinander werden Triethylamin (230 µl, 6.4 mmol), Triphenylphosphin (polymergebunden, 1.1 g, 3.2 mmol) und Dibromtetrachlorethan (1.04 g, 3.2 mmol) zugegeben. Das Gemisch wird bei 0°C bis zum vollständigen Umsatz gerührt (1h), in Dichlormethan aufgenommen und mit Wasser gewaschen. Nach Enfernen des Lösungsmittels am Rotationsverdampfer wird der Rückstand flashchromatographisch (Isohexan/Esssigester 4/1 - 2/1) gereinigt. Man erhält **11** als farbloses Öl (0.79 g, 1.47 mmol, 92 %).
DC [Toluol/Essigester: 1/1]: R_{f} = 0.85 - ¹H-NMR (300 MHz, CDCl₃): d = 1.15 (d, 3 H, J_{6,5} = 6.9 Hz, 6-H^{Fuc}), 1.56-2.00 (m, 3H), 3.40 (m, 2H), 3.78 (m, 3 H), 3.80-4.00 (m, 3H), 4.40-4.80 (m, 6 H), 7.30-7.40 (m, 15 H).

### Beispiel 2

### Vorschrift zur Darstellung der Carbamate 12a - d

Der Alkohol 10 wird in Dichlormethan gelöst (10 ml/mmol) und mit Triethylamin (1.1 eq.) versetzt. Bei 0°C wird Chlorameisensäure-p-nitrophenylester (1.3 eq.) und eine katalytische Menge DMAP zugegeben. Die Lösung wird über Nacht gerührt, mit DIPEA (3 eq.) und der Aminkomponente (1.5 eq.) versetzt. Die Lösung wird weitere 10 h bei Raumtemperatur gerührt, in Dichlormethan aufgenommen und mit gesättigter Natriumhydrogencarbonatlösung gewaschen. Nach Enfernen des Lösungsmittels am Rotationsverdampfer wird der Rückstand in MeOH/Dioxan/AcoH (5/1/1) gelöst und mit Pd/C (10 %ig) als Katalysator (10 h) hydriert. Nach Abfiltrieren des Katalysators und Entfernen des Lösungsmittels wird der ölige Rückstand in 1M Natronlauge (3-5 ml) aufgenommen und bis zur vollständigen Verseifung (2 h) gerührt. Das Gemisch wird chromatographisch (Bakerbond, Kieselgel RP 18, 40 µm, Methanol/Wasser 10/90 - 90/10) gereinigt.
12aDC [Essigester/Methanol/Ameisensäure/Wasser 5/3/0.5/1]: R_{f} = 0.30 - ¹H-NMR (300 MHz, D₂O): d = 1.05 (d, 3 H, J_{6,5} = 6.9 Hz, 6-H^{Fuc}), 1.60.1.95 (m, 2H), 2.96 (m, 1H), 3.78-10 (m, 8H), 4.58 (m, 1H).
12b DC [Essigester/Methanol/Ameisensäure/Wasser 5/3/0.5/1]: R_{f} = 0.35 - ¹H-NMR (300 MHz, D₂O): d = 1.05 (d, 3 H, J_{6,5} = 6.9 Hz, 6-H^{Fuc}), 1.10-1.80 (m, 10 H), 3.00 (m, 2 H), 3.57-4.05 (8 H).
12c DC [Essigester/Methanol/Ameisensäure/Wasser 5/3/0.5/1]: R_{f} = 0.25 - ¹H-NMR (300 MHz, D₂O): d = 1.5 (d, 3 H, J_{6,5} = 6.9 Hz, 6-H^{Fuc}), 1.25-1.80 (m, 7 H), 2.4 (m, 1H), 2.80 (m, 2 H), 3.60-4.05 (m, 9 H).
12d DC [Essigester/Methanol/Ameisensäure/Wasser 5/3/0.5/1]: R_{f} = 0.20 - ¹H-NMR (300 MHz, D₂O): d = 1.5 (d, 3 H, J_{6,5} = 6.9 Hz, 6-H^{Fuc}), 1.80-2.05 (m, 4 H), 3.60-4.50 (m, 16 H).

### Beispiel 3

### Vorschrift zur Darstellung der Amine 13a,b

Zu einer Lösung des Bromids 11 in Dichlormethan (3 ml/mmol) wird das Amin (1 eq.), Triethylamin (2 eq.) und eine katalytische Menge DMAP gegeben. Das Gemisch wird bei Raumtemperatur bis zur vollständigen Umsetzung gerührt, in Dichlormethan aufgenommen und mit Wasser gewaschen. Nach Enfernen des Lösungsmittels am Rotationsverdampfer wird der Rückstand flashchromatographisch ( Kieselgel, Isohexan/Essigsäureethylester 4/1-1/1) gereinigt. Der Rückstand wird in MeOH/Dioxan/AcoH (5/1/1) gelöst und mit Pd/C (10 %ig) als Katalysator (10 h) hydriert. Nach Abfiltrieren des Katalysators und Entfernen des Lösungsmittels wird der ölige Rückstand in 1M Natronlauge (3-5 ml) aufgenommen und bis zur vollständigen Verseifung gerührt. Das Gemisch wird chromatographisch (Bakerbond, Kieselgel RP 18, 40 µm, Methanol/Wasser 10/90 - 90/10) gereinigt.
13a DC [Essigester/Methanol/Ameisensäure/Wasser 5/3/0.5/1]: R_{f} = 0.25;
13b DC [Essigester/Methanol/Ameisensäure/Wasser 5/3/0.5/1]: R_{f} = 0.20.

### Beispiel 4

### Mannosederivate

Analog zu **8** wird das Mannosederivat **14** als α/β Gemisch über 3 Stufen in 62 % Ausbeute dargestellt.
DC [Dichlormethan/Methanol: 10/1]: R_{f} = 0.30 - ¹H-NMR (300 MHz, D₂O): d = 2.19-2.43 (m, 2H), 3.60-4.00 (m, 7 H), 5.05 (m, 2 H), 5.62-5.80 (m, 1H).

Durch Benzylierung von **14** analog **9** wird die Allylverbindung **15** dargestellt. **15** kann auch direkt durch Allylierung der entsprechenden Benzylvorstufe dargestellt werden.
DC [Toluol/Essigester: 5/1]: R_{f} = 0.45 - ¹H-NMR (300 MHz, CDCl₃): d = 2.35 m, 2 H), 3.60-4.20 (m, 6 H), 4.50-4.60 (m, 8H), 4.72 (m, 1H), 5.00 (m, 2H), 5.78 (m, 1H), 7.20-7.40 (m, 20 H).

Hydroborierung von **15** liefert **16**.
DC [Toluol/Essigester: 1/1]: R_{f} = 0.25 - ¹H-NMR (300 MHz, CDCl₃): d = 1.60 (m, 4 H), 3.58-4.05 (m, 9 H), 3.50-4.05 (m, 8H).

Analog zu dem Fucosederivat **10** werden aus **16** die Derivate **17a-c** und **18a,b** aufgebaut.
**17a**DC [Essigester/Methanol/Ameisensäure/Wasser 5/3/0.5/1]: R_{f} = 0.25 - ¹H-NMR (300 MHz, D₂O): d = 1.40-1.93 (m, 9H), 2.5 (m, 1H), 2.80-3.00 (m, 2H), 3.40-4.12 (m, 10H).

### Asparaginderivat

**17b**DC [Essigester/Methanol/Ameisensäure/Wasser 5/3/0.5/1]: R_{f} = 0.30 - ¹H-NMR (300 MHz, D₂O): d = 1.40-1.75 (m, 4H), 3.20 (m, 1H), 3.40-3.82 (m, 10H).

### Lysinderivat

**17c**DC [Essigester/Methanol/Ameisensäure/Wasser 5/3/0.5/1]: R_{f} = 0.30 - ¹H-NMR (300 MHz, D₂O): d = 1.30-1.78 (m, 7 H), 3.35-3.80 (m, 15H).

### Piperidinderivat

**18** DC [Essigester/Methanol/Ameisensäure/Wasser 5/3/0.5/1]: R_{f} = 0.30 - ¹H-NMR (300 MHz, D₂O): d = 1.38-1.50 (m, 1H), 1.60-1.85 (m, 5H), 1.92-2.04 (m, 2H), 2.30-2.40 (m, 1H), 2.80-3.15 (m, 4 H), 3.22-3.88 (m, 9H).

### Beispiel 5

### Ribosederivate

1-O-acetyl-2,3,5-tri-O-benzoyl-β-D-ribofuranose (1.0 g, 2.0 mmol) wird unter Argon in Acetonitril (10 ml) gelöst und auf -10 °C abgekühlt. Nach Zugabe von Allylsilan (0.48 ml, 3.0 mmol) und Bortrifluorid - Diethylether Komplex (0.25 ml, 0.2 mmol) wird das Gemisch 1 h bei -10 °C gerührt. Nach Erwärmen auf Raumtemperatur wird weitere 2 h gerührt. Die Lösung wird auf gesättigte Natriumhydrogencarbonatlösung gegeben und mit Essigester extrahiert. Das Lösungsmittel wird am Rotationsverdampfer entfernt. Der verbleibende Rückstand wird in Dichlormethan aufgenommen und über eine kurze Kieselgelsäule filtriert. Nach Entfernen des Lösungsmittels erhält man die Allylverbindung (a/β 4/1) als gelbes Öl (0.798 g, 76 %).
DC [Hexan/Essigsäureethylester : 1/1]: R_{f} = 0.60 - ¹H-NMR (300 MHz, CDCl₃): d = 2.51 (m, 2 H), 4.20-6.05 (m, 8 H), 7.10-8.35 (m, 15 H).

## Patentansprüche

1. Verbindung der Formel I 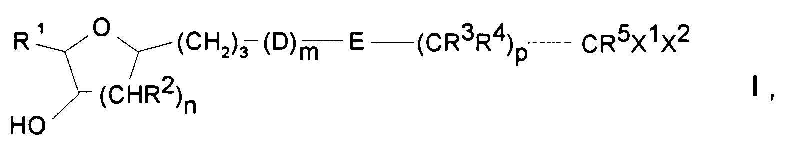 worin bedeuten
R¹ -H, -CH₃, oder -CH₂OH,
R² -H oder -OH,
R^{3,} R⁴ und R⁵ unabhängig voneinander -H, C₁-C₄-Alkyl oder -OH,
R⁶, R⁷, R⁸, R⁹ und R¹⁰ unabhängig voneinander -H oder C₁-6₄-Alkyl,
D -O-C(O)-, -C(O)- oder -NR⁶-C(O)-,
E -CR⁷R⁸-, -NR⁷-, einen Stickstoffheterozykus der Formel 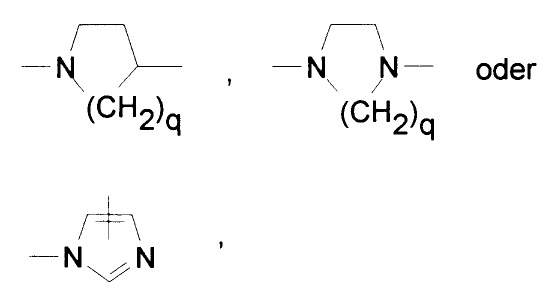
n 1 oder 2,
m 0 oder 1,
p eine ganze Zahl von 0 bis 10,
q 1 oder 2 und
X¹ und X² unabhängig voneinander -H, -COOR⁹, -NR⁹R¹⁰, -OH, -OSO₃H, -CH₂COOR⁹, -CH₂OSO₃H oder gemeinsam =O.

2. Verbindung nach Anspruch 1, dadurch gekennzeichntet, daß m 1 ist und D -O-C(O)- bedeutet.

3. Verbindung nach Anspruch 1, dadurch gekennzeichnet, daß m 0 ist.

4. Verbindung nach Anspruch 2 oder 3, dadurch gekennzeichntet, daß
E -NR⁷-,
R⁷ -H,
p 0,
R⁵ -H
X¹ -COOR⁹,
X² -CH₂COOR⁹ und
R⁹ -H bedeuten.

5. Verbindung nach Anspruch 2 oder 3, dadurch gekennzeichntet, daß
E -NR⁷-,
R⁷ -H,
p 4,
R³ und R⁴ -H
R⁵ -H
X¹ -COOR⁹,
X² -NR⁹R¹⁰ und
R⁹ und R¹⁰ -H bedeuten.

6. Verbindung nach Anspruch 2 oder 3, dadurch gekennzeichntet, daß
E 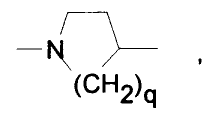
q 2,
p 0,
R⁵ -OH und
X¹ und X² gemeinsam = O bedeuten.

7. Verbindung nach Anspruch 2 oder 3, dadurch gekennzeichntet, daß
E 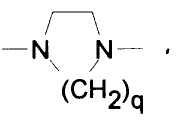
q 2,
p 0,
R⁵ und R⁹ -H und
X¹ und X² -COOR⁹ bedeuten.

8. Verbindung nach einem der Ansprüche 1 bis 7, dadurch gekennzeichnet, daß n 2 ist und R² -OH bedeutet.

9. Verbindung nach Anspruch 8, dadurch gekennzeichnet, daß R¹ -CH₃ bedeutet.

10. Verbindung nach Anspruch 9, dadurch gekennzeichnet, daß der Oxazyklus in Formel I 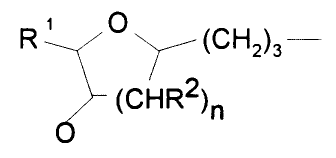 die Konfiguration eines L-Fucosylrests aufweist.

11. Verbindung nach Anspruch 8, dadurch gekennzeichnet, daß R¹ -CH₂OH bedeutet.

12. Verbindung nach Anspruch 11, dadurch gekennzeichnet, daß der Oxazyklus in Formel I 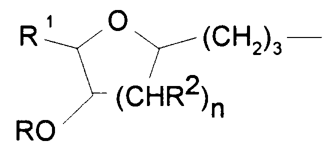 die Konfiguration eines D-Mannosylrests aufweist.

13. Verbindung nach einem der Ansprüche 1 bis 7, dadurch gekennzeichnet, daß n 1, R¹ -CH₂OH und R² -OH bedeuten.

14. Verbindung nach Anspruch 13, dadurch gekennzeichnet, daß der Oxazyklus in Formel I 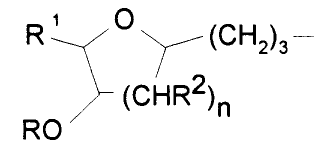 die Konfiguration eines Ribosylrests aufweist.

15. Verfahren zur Herstellung einer Verbindung gemäß einem der Ansprüche 1 bis 14, dadurch gekennzeichnet, daß man eine Verbindung der Formel II 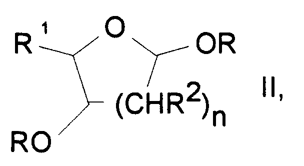 worin R¹, R² und n die genannten Bedeutungen haben, wobei R¹ und R² notwendigenfalls mit Schuzgruppen versehen sind, und R eine Acetylgruppe bedeutet, unter der Wirkung eines Lewissäurekatalysators in einem geeigneten Lösungsmittel mit einem Allylsilan zu einer Verbindung der Formel III 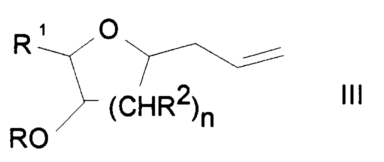 umsetzt, in welcher sämtliche Variablen die genannten Bedeutungen haben, worauf nach Umwandlung sämtlicher Schutzgruppen in Benzylschutzgruppen unter Funktionalisierung der allylischen Doppelbindung eine Verbindung der Formel IV 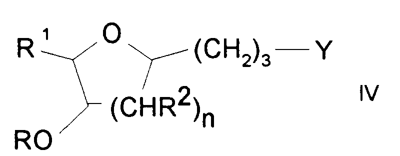 hergestellt wird, in welcher die Variblen R¹ und R² die genannten Bedeutungen haben und notwendigenfalls mit Schutzgruppen versehen sind, R eine Benzylgruppe bedeutet und Y Halogen, -NHR⁶ oder eine aktivierte OH-Gruppe darstellt, wonach die Verbindung der Formel IV mit einer Verbindung der Formel V
X―E―(CR³R⁴)ₚ― CR⁵X¹X² V
umgesetzt wird, inwelcher X -H oder -COOH bedeutet und sämtliche übrigen Variablen die genannten Bedeutungen haben, worauf man nach Abspaltung sämtlicher Schutzgruppen eine Verbindung der Formel I gemäß einem der Ansprüche 1 bis 13 erhält.

16. Arzneimittel enthaltend wenigstens eine Verbindung gemäß einem der Ansprüche 1 bis 14.

17. Verwendung einer Verbindung gemäß einem der Ansprüche 1 bis 14 für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen.

18. Verbindung gemäß einem der Ansprüche 1 bis 14 zur Verwendung als Arzneimittel.
